# EUROPEAN PATENT SPECIFICATION

(11) **EP 4 157 305 B1**
(45) Date of publication and mention of the grant of the patent: **02.07.2025**
(21) Application number: 21726425.8
(22) Date of filing: 24.05.2021
(51) Int. Cl.: A61K 38/00, A61P 9/10, C07K 14/47

(54) **PI3K GAMMA INHIBITOR PEPTIDE FOR TREATMENT OF FIBROPROLIFERATIVE VASCULAR DISEASES**
PI3K GAMMA INHIBITOR PEPTID FÜR DIE BEHANDLUNG VON FIBROPROLIFERATIVEN VASKULÄREN ERKRANKUNGEN
INHIBITEUR PEPTIDIQUE DÉRIVÉ DE PI3K GAMMA POUR LE TRAITEMENT DES MALADIES FIBROPROLIFERATIVES VASCULAIRES DISEASES

(30) Priority: 25.05.2020 IT 202000012259
(43) Date of publication of application: 05.04.2023
(73) Proprietor: Kither Biotech S.r.l., 10126 Torino (IT)
(72) Inventor: GHIGO, Alessandra, 12038 Savigliano (Cuneo) (IT); HIRSCH, Emilio, 10125 Torino (IT)
(74) Representative: Buzzi, Notaro & Antonielli d'Oulx S.p.A.
(86) International application number: PCT/EP2021/063757
(87) International publication number: WO 2021/239662

(56) References cited:
- WO-A1-2016/103176
- QIHONG YU ET AL: "PI3K[gamma] (Phosphoinositide 3-Kinase [gamma]) Regulates Vascular Smooth Muscle Cell Phenotypic Modulation and Neointimal Formation Through CREB (Cyclic AMP-Response Element Binding Protein)/YAP (Yes-Associated Protein) Signaling", ARTERIOSCLEROSIS, THROMBOSIS, AND VASCULAR BIOLOGY, vol. 39, no. 3, 1 March 2019 (2019-03-01), pages e91 - e105, XP055766148, ISSN: 1079-5642, DOI: 10.1161/ATVBAHA.118.312212
- QIHONG YU ET AL: "PI3K[gamma] promotes vascular smooth muscle cell phenotypic modulation and transplant arteriosclerosis via a SOX9-dependent mechanism", EBIOMEDICINE, vol. 36, 1 October 2018 (2018-10-01), NL, pages 39 - 53, XP055766225, ISSN: 2352-3964, DOI: 10.1016/j.ebiom.2018.09.013
- NEEDLEMAN S B ET AL: "A general method applicable to the search for similarities in the amino acid sequence of two proteins", JOURNAL OF MOLECULAR BIOLOGY, ACADEMIC PRESS, UNITED KINGDOM, vol. 48, no. 3, 28 March 1970 (1970-03-28), pages 443 - 453, XP024011703, ISSN: 0022-2836, [retrieved on 19700328], DOI: 10.1016/0022-2836(70)90057-4

## Description

### Field of the invention

The present description concerns a new strategy for the therapeutic treatment of fibroproliferative vascular diseases.

### Background art

Cardiovascular diseases represent a significant part of morbidity and mortality worldwide. These pathologies often originate from vascular dysfunction and occlusion, occurring even after interventional strategies used in the treatment of atherosclerosis. For instance, intimal hyperplasia (IH) ensues from atherosclerotic plaque development but also after transluminal angioplasty and stent positioning, a standard treatment of atherosclerosis. IH consists in a complex multifactorial response of the vessel wall resulting in the formation of a multicellular layer within the arterial lumen. It involves inflammatory and fibroproliferative phenomena due to endothelial injury and subsequent vascular smooth muscle cell (VSMC) dedifferentiation, leading to their over-proliferation and migration. Together, these processes end up with intimal thickening and a reduction in blood flow, which can have dramatic consequences. Thus, understanding mechanisms of VSMC proliferation is critical for the development of new therapeutic strategies to prevent IH [1, 2].

Among the signaling molecules involved in VSMC proliferation, the second messenger 3'-5' cyclic adenosine monophosphate (cAMP) plays a major role. In VSMC, elevated cAMP levels inhibit mitogen-stimulated proliferation both *in vitro* and *in vivo* [3-6]. Consistently, cAMP has been found to have a positive role in reducing intimal thickening in several models of vascular injury. Moreover, cAMP signaling is affected in a large variety of vascular pathologies including atherosclerosis or restenosis after angioplasty. cAMP signals mainly depend on its localized production by adenylyl cyclases (AC) and its degradation by cyclic nucleotide phosphodiesterases (PDE) [7]. In VSMC, PDE3A, PDE4 and PDE1C have been implicated in the regulation of VSMC proliferation and IH [3, 8, 9]. Interestingly, it has been shown that cAMP production is linked to an increase of PDE4 activity and upregulated expression of the PDE4D isoform in both contractile and synthetic phenotypes [10]. Consistently, numerous studies have been performed using PDE inhibitors in various arterial disease models [3].

In this context, phosphoinositide 3-kinase gamma (PI3Kγ), a lipid kinase involved in immune regulation of cardiovascular diseases, has been described to regulate cAMP levels in cardiomyocytes through a kinase-independent mechanism. While, on the one hand, PI3Kγ can generate 3-phosphoinositides lipid second messengers as a kinase, on the other hand, it can act as a "A-kinase anchoring protein" (AKAP) associating, within the same macromolecular complex, with cAMP-dependent kinase, also known as protein kinase A (PKA), and phosphodiesterase 3 and 4 (PDE3 and PDE4), PKA-controlled enzymes responsible for cAMP degradation. In line with this view, the catalytic subunit of the PI3Kγ holoenzyme (p110γ) interacts with PKA and participates in a negative feedback loop, leading to PDE3 and PDE4 activation and eventually to increased cAMP degradation.

In cardiac cells, PI3Kγ restrains cAMP elevation in response to beta-adrenergic stimulation, ultimately reducing cardiac contractility [11]. On the contrary, PI3Ky-mediated regulation of cAMP levels in airway smooth muscle cells promotes contraction, leading to bronchoconstriction. Accordingly, the inhibition of the kinase-independent function of PI3Kγ in airway smooth muscles with a cell-permeable competing peptide promotes bronchorelaxation [12].

### Summary of the invention

The object of this disclosure is to provide a new strategy for the treatment/prevention/delay of onset of fibroproliferative vascular diseases.

According to the invention, the above object is achieved thanks to the subject matter recalled specifically in the ensuing claims, which are understood as forming an integral part of this disclosure.

The present invention concerns a fusion peptide, and a pharmaceutical composition containing a fusion peptide for use in treating, preventing and/or delaying onset of a fibroproliferative vascular disease, wherein the fusion peptide comprises:
(a) an amino acid sequence as defined in SEQ ID No.: 1 or a related homolog having at least 85%, preferably at least 90%, more preferably at least 95% identity with SEQ ID No.: 1 and having the ability of the sequence SEQ ID No.: 1 to inhibit the kinase-independent function of PI3Kγ, and
(b) a peptide having the ability to penetrate a cell.

Arterial remodeling found in hypertension and intimal hyperplasia involves inflammation and disrupted flow, both contributing to smooth muscle cells dedifferentiation and proliferation. In the present application, the inventors identified for the first time a kinase-independent key role of non-hematopoietic phosphoinositide 3-kinase gamma (PI3Kγ) in the vascular wall during intimal hyperplasia using mouse models deleted for PI3Kγ or expressing a Kinase Dead version of the enzyme. Moreover, the inventors found that absence of PI3Kγ in VSMC leads to a modulation of cell proliferation linked to an increase in intracellular cAMP levels. Real time analysis of cAMP dynamics reveals that PI3Kγ modulates the degradation of cAMP independently of its kinase activity in primary VSMC through the regulation of phosphodiesterase (PDE) 4 enzymes. The present invention thus concerns the therapeutic application of the N-terminal competing peptide of PI3Kγ (having the amino acid sequence set forth in SEQ ID No.: 1) or a corresponding homolog that is able to block primary VSMC proliferation. These data provide evidence for a kinase-independent role of PI3Kγ in arterial remodeling and reveal novel strategies targeting the docking function of PI3Kγ for the treatment/prevention/delay of onset of fibroproliferative vascular diseases.

### Brief description of the drawings

The invention will now be described in detail, purely by way of an illustrative and non-limiting example and, with reference to the accompanying drawings, wherein:

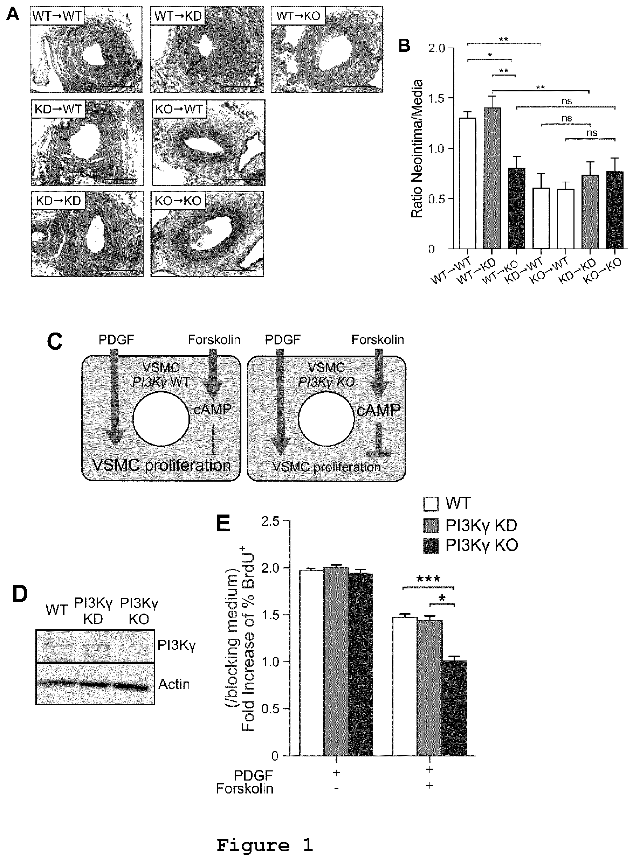

- **Figure 1****: PI3Kγ controls IH and VSMC proliferation**
   **A.** Representatives cross sections of injured femoral arteries stained with Masson Trichrome from the indicated hematopoietic chimeras. Scale bars = 100 µM. Irradiated WT, PI3Kγ KD (KD) and PI3Kγ KO recipient mice were transplanted with bone marrow from the indicated donors to obtain the indicated chimeras. Irradiated WT mice were transplanted with bone marrow from WT, PI3Kγ KD or PI3Kγ KO to obtain the following hematopoietic chimeras: WT>WT, KD>WT and KO>WT. Irradiated KD recipient mice were transplanted with bone marrow from WT or PI3Kγ KD donor mice (WT>KD; KD>KD). Irradiated KO recipient mice were transplanted with bone marrow from WT or KO donors (WT>KO and KO>KO). **B.** Quantitative analysis of intima/media ratios of indicated hematopoietic chimeras. Histogram represents the neointima/media ratio for each chimera (n > 6 mice for each group). Data are presented as mean ± SEM and were compared using a one-way ANOVA test. **C.** Schematic representation of the experimental model used to investigate PI3Kγ implication in the control of SMC proliferation. **D.** Western blot of PI3Kγ expression in each genotype. **E.** Proliferation rate measured by BrdU incorporation and expressed as fold increase compared to control of primary VSMC from WT, PI3Kγ KO or PI3Kγ KD aorta incubated 24h with blocking medium, or treated with 25 ng/ml PDGF, with or without addition of 25 µM forskolin. (5<n<12 cultures for each genotype). Data are presented as mean ± SEM and were compared using a one-way ANOVA test.

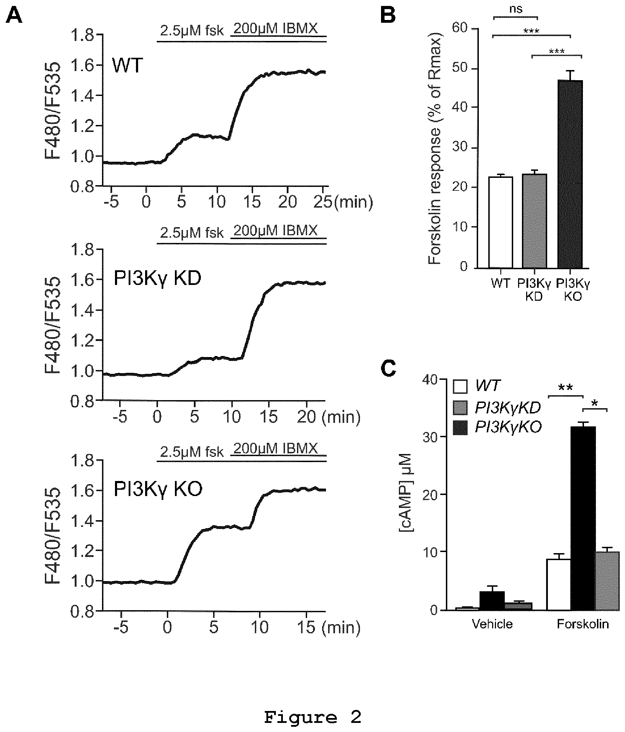

- **Figure 2****: PI3Kγ controls cAMP dynamics independently of its kinase activity in VSMC**
   **A.** Left panel: Average changes in intracellular cAMP concentration ([cAMP]ᵢ) in primary VSMC from WT (top), PI3Kγ KO (middle) and PI3Kγ-KD (bottom) mice and expressing the ^{T}Epac^{VV} biosensor. The black line represents the average F480/F535 emission ratio over time in response to 2.5 µM forskolin (fsk) stimulation and fsk (2.5µM) + IBMX (200 µM) treatment. **B.** Histograms representing the mean values for forskolin responses. Results are expressed as a percentage of the maximal ratio change (% of Rmax(IBMX response)) determined by the final application of forskolin (2.5µM) + IBMX (200 µM). Data shown are the means ± SEM. (n= 5 mice). Data are presented as mean ± SEM and were compared using a Kruskal-Wallis test. **C.** Homogenous time-resolved fluorescence (HTRF) dosage of intracellular cAMP from WT, PI3Kγ KO or PI3Kγ KD primary VSMC after 30 min treatment with vehicle or 0.5 µM Forskolin.

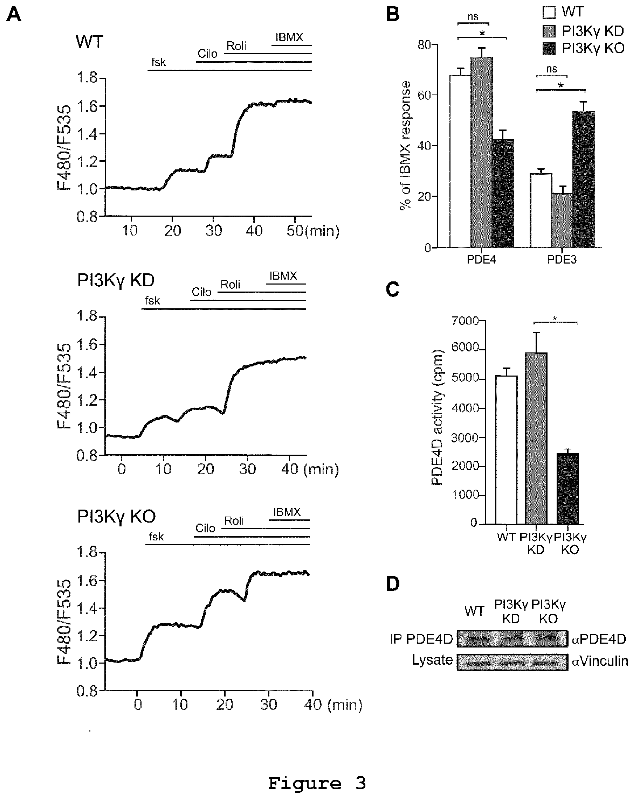

- **Figure 3****: PI3Kγ controls cAMP levels via PDE4 in VSMC**
   **A.** Average changes in [cAMP]ᵢ in primary VSMC from WT (top), PI3Kγ KO (middle) and PI3Kγ-KD (bottom) mice and expressing the ^{T}Epac^{VV} biosensor. The trace indicates the average F480/F535 emission ratio over time in response to 2.5 µM forskolin, cilostamide (1 µM), rolipram (1 µM) and forskolin (2.5 µM) + IBMX (200 µM) treatment. **B.** Histograms representing the mean values for rolipram and cilostamide responses. Results are expressed as a percentage of the maximal ratio change (% of Rmax (IBMX response)) determined by the final application of forskolin (2.5 µM) + IBMX (200 µM). Data are presented as mean ± SEM (n>22 with 4 different mice) and were compared using a Kruskal-Wallis test. **C.** Phosphodiesterase activity detected in PDE4D immunoprecipitates from primary VSMC. Data shown are the means ± SEM and compared using one-way ANOVA followed by Bonferroni post-hoc test (n=3). **D.** Representative Western blot of PDE4D immunoprecipitates.

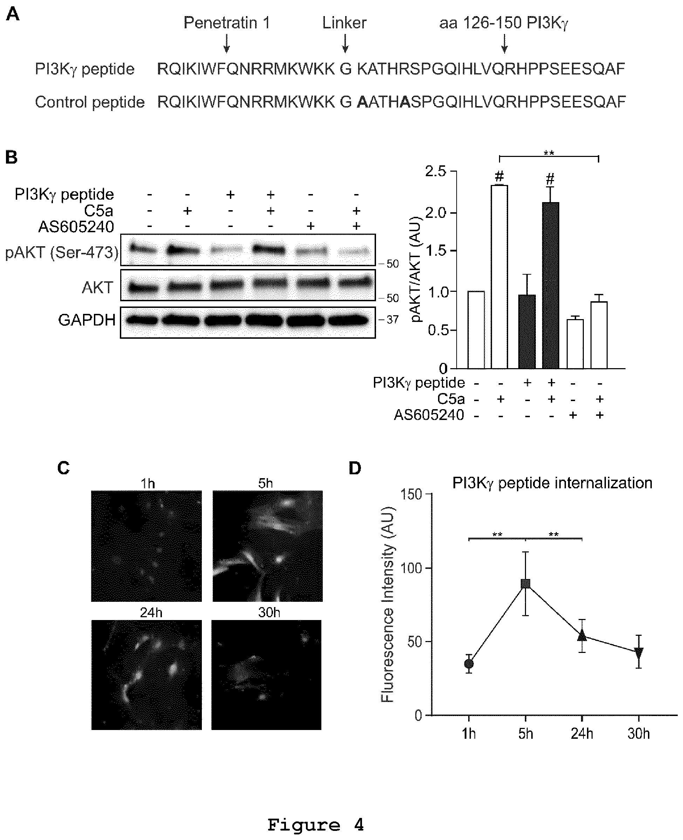

- **Figure 4****: A permeant N terminal peptide of PI3Kγ selectively interferes with the docking activity of PI3Kγ**
   **A.** Schematic representation of the cell-permeable PI3Kγ competing peptide. The 126-150 region of PI3Kγ was fused to the cell-penetrating peptide Penetratin 1 (P1). A control peptide was generated by insertion of two point mutations (K>A and R>A at position 1 and 5 of the PI3Kγ sequence, respectively). **B.** RAW macrophages were pre-treated with vehicle, the PI3Kγ peptide (10 µM) or the PI3Kγ kinase inhibitor AS605240 (1 µM) for 1 hour and then with C5a (50 µM) for 5 minutes. Representative Western blot images of phospho-AKT (Ser-473) and total AKT (left) and ratio quantification (right) are shown. GAPDH was used as loading control. n=3. **C.** Incorporation by primary VSMC of a FITC-labelled PI3Kγ peptide at 1h, 5h, 24h and 30h after incubation. **D.** Quantification of FITC-associated fluorescence intensity inside VSMC area.

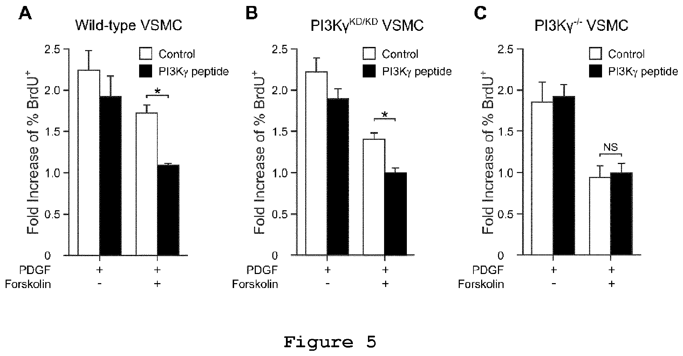

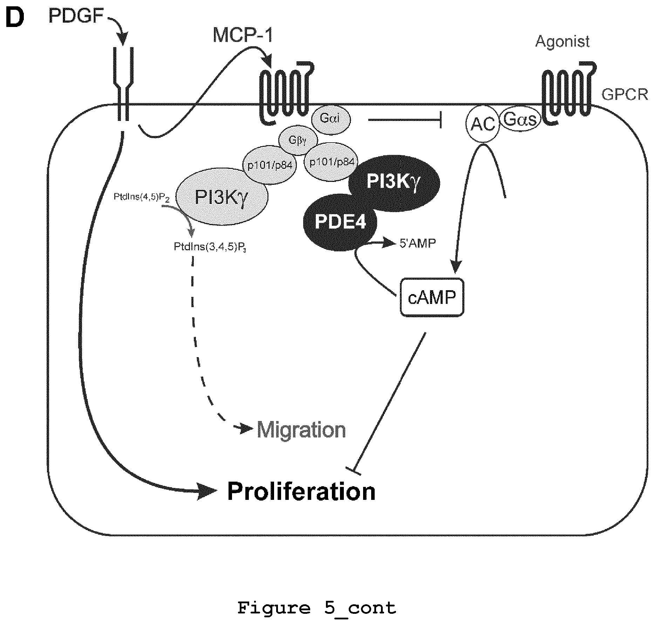

- **Figure 5****: A permeant N-terminal peptide of** PI3K**γ reduces VSMC proliferation**
   **A, B, C.** Proliferation rate measured by BrdU incorporation and expressed as fold increase compared to control of primary VSMC from WT (**A**), PI3Kγ KD (**B**) or PI3Kγ KO (**C**) aorta incubated for 24h with 25 ng/ml PDGF with or without addition of 25 µM forskolin, in the presence of 25 µM of control (white bars) or blocking peptide (black bars). BrdU was added during the last 18h (n=8 cultures for each genotype). Data are presented as mean ± SEM and were compared using a one-way ANOVA test. **D.** Schematic model of PI3Kγ implication in VSMC proliferation. VSMC proliferation is induced by PDGF stimulation. Inventors' previous results, represented in gray, demonstrate that autocrine secretion of MCP1 recruits and activates PI3Kγ through GPCR signaling to control cell migration after PDGF stimulation [13]. This PI3Kγ recruitment could also concentrate phosphodiesterases (PDE) that counterbalance cAMP production by adenylate cyclase (AC) induced by other signals. By these mechanisms, PI3Kγ acts as an amplifier of VSMC synthetic transition.

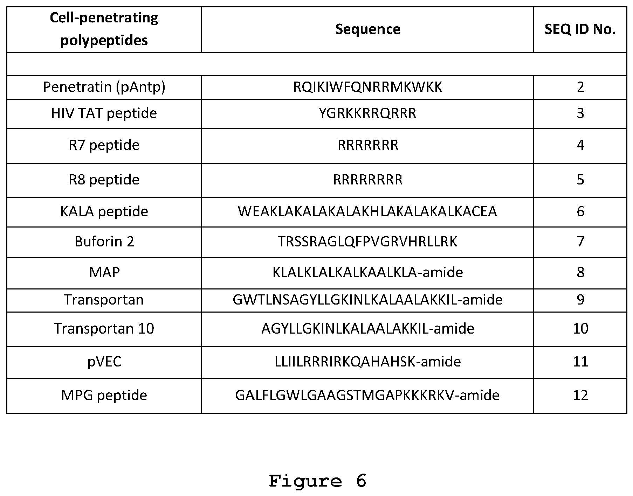

- **Figure 6****: Amino acid sequences of peptides having the ability to penetrate a cell membrane.**

### Detailed description of the invention

In the description that follows, numerous specific details are given to provide a thorough understanding of the embodiments.

The headings provided herein are for convenience only and do not interpret the scope or meaning of the embodiments.

In one embodiment, the present invention concerns a fusion peptide for use in treating, preventing and/or delaying onset of a fibroproliferative vascular disease, wherein the fusion peptide comprises:
(a) an amino acid sequence as defined in SEQ ID No.: 1 or a related homolog having at least 85%, preferably at least 90%, more preferably at least 95% identity with SEQ ID No.: 1 and having the ability of the sequence SEQ ID No.: 1 to inhibit the kinase-independent function of PI3Kγ, and
(b) a peptide having the ability to penetrate a cell.

In a further embodiment, the peptide having the ability to penetrate a cell is selected from the sequences specified in SEQ ID No.: 2 to 12, preferably SEQ ID No.: 2 to 5, more preferably SEQ ID No.: 2.

In one embodiment, the fusion peptide can comprise a linker, preferably an amino acid linker, that allows the linking of the amino acid sequence as defined in SEQ ID No.: 1 or its homolog with the peptide having the ability to penetrate a cell. The presence of the linker is however not mandatory.

In a still further embodiment, the peptide having the ability to penetrate a cell is linked to the C-terminal or the N-terminal of SEQ ID No.: 1 or its homologs.

In a further embodiment, the fusion peptide has an amino acid sequence as set forth in SEQ ID No.: 14.

In a further embodiment, the fusion peptide as defined above is suitable for the treatment/prevention/delay of onset of a fibroproliferative vascular disease selected from restenosis, hypertension, pulmonary hypertension, preferably pulmonary arterial hypertension, atherosclerosis and plaque rupture caused by intimal thickening.

In a different embodiment, the present invention concerns a pharmaceutical composition for use in treating, preventing and/or delaying onset of a fibroproliferative vascular disease containing a fusion peptide, and a pharmaceutically acceptable vehicle, wherein the fusion peptide comprises (a) an amino acid sequence as defined in SEQ ID No.: 1 or a related homolog having at least 85%, preferably at least 90%, more preferably at least 95% identity with SEQ ID No.: 1 and having the ability of the sequence SEQ ID No.: 1 to inhibit the kinase-independent function of PI3Kγ, and (b) a peptide having the ability to penetrate a cell.

In one embodiment, the fusion peptide contained in the pharmaceutical composition comprises a peptide having the ability to penetrate a cell selected from the sequences specified in SEQ ID No.: 2 to 12, preferably SEQ ID No.: 2 to 5, more preferably SEQ ID No.: 2.

In a preferred embodiment, the fusion peptide contained in the pharmaceutical composition comprises a peptide having the ability to penetrate a cell, wherein such a peptide is linked to the C-terminal or the N-terminal of SEQ ID No.: 1 or its homologs.

In a preferred embodiment, the fusion peptide contained in the pharmaceutical composition can comprise a linker, preferably an amino acid linker, that allows the linking of the amino acid sequence as defined in SEQ ID No.: 1 or its homolog with the peptide having the ability to penetrate a cell. The presence of the linker is however not mandatory.

In a further embodiment, the pharmaceutical composition comprises a fusion peptide having an amino acid sequence as set forth in SEQ ID No.: 14.

In a preferred embodiment, the pharmaceutical composition is suitable for the treatment/prevention/delay of onset of a fibroproliferative vascular disease selected from restenosis, hypertension, pulmonary hypertension, preferably pulmonary arterial hypertension, atherosclerosis and plaque rupture caused by intimal thickening.

Also disclosed but not claimed is a method for treating/preventing/delaying onset of a fibroproliferative vascular disease comprising administering to a patient in need thereof at least one fusion peptide in an amount sufficient to carry out said treatment, wherein the fusion peptide comprises:
a) the amino acid sequence as defined in SEQ ID No.: 1 or a related homolog having at least 85% similarity with SEQ ID No.: 1 and having the ability of the sequence SEQ ID No.: 1 to inhibit the kinase-independent function of PI3Kγ, and
b) a peptide having the ability to penetrate a cell.

In various diseases like atherosclerosis [14] and pulmonary arterial hypertension [15, 16], intimal vascular smooth muscle cells (VSMC) abnormally proliferate, eventually triggering intimal hyperplasia (IH) leading to vessel occlusion. Angioplasty and metal stent implantation open vessels narrowed by such VSMC overgrowth, but this treatment fails if VSMC continue to expand and trigger restenosis. Similarly, vein by-pass grafts are frequently unsuccessful owing to the growth of VSMC into a new occlusive intimal layer. Thus, pharmacological reduction of VSMC proliferation is of critical importance in the treatment of vascular occlusion due to IH.

A large body of literature suggests that intracellular increase of the signaling molecule cAMP plays an important role in both the maintenance of VSMC quiescence in healthy vessels and the reduction of restenosis in diseased vascular districts [6]. Small molecule inhibitors of phosphodiesterases (PDE), the cAMP hydrolyzing enzymes, have an anti-proliferative effect on VSMC, and drugs like cilostazol may have potential for clinical therapeutic use for the prevention of restenosis following arterial intervention [17]. The difficulty of limiting systemic exposure to such drugs, eventually leading to unwanted side effects, like cardiac arrythmia, prevented large-scale adoption of such interventions. In addition, PDEs are a large family of isoenzymes, classified in 11 groups [6], and the lack of isoform-selective PDE inhibitors, especially within members of the same family, further restrained the application of safe and effective cAMP elevation, in the treatment of IH [6].

The present results indicated that a cell permeable peptide containing residues between 126 and 150 of the N-terminal domain of the protein PI3Kγ (SEQ ID No.: 1) can selectively increase cAMP in VSMC by disturbing the function of a specific PDE isoform, PDE4D. This peptide (named KIT2014; [12]) disrupts the interaction between PI3Kγ and PKA, delocalizing PKA and reducing its ability to activate selected cell-type specific PDE isoforms [18]. The amino acid sequence of PI3Kγ found in KIT2014 has been previously shown to decrease PDE3 activity causing arrythmia *in vitro* in cardiomyocytes [19]. Moreover, this peptide has been shown to disrupt the PI3Kγ/PKA complex in airway smooth muscles, leading to PDE4B/D inhibition and bronchorelaxation [12]. VSMC, though being similar to airway smooth muscles, are not superimposable in terms of gene expression and cell biology [20, 21], thus complicating predictions of peptide efficacy in these cells. The present results demonstrate that, when conjugated with a cell-penetrating peptide like Penetratin 1, this peptide sequence efficiently enters VSMC and increases their cAMP levels.

Whether KIT2014 was able to trigger cAMP-dependent proliferative arrest in VSMC was impossible to predict from prior art. Consensus on cAMP way of action evidences complex subcellular signaling compartmentalization due to the existence, in the same cell, of different pools of cAMP with distinct functions [22]. Inventors' previous finding demonstrated that in cardiomyocytes and in airway smooth muscles KIT2014 regulates pools of cAMP which control cardiac contraction and bronchorelaxation, respectively. Data reported here confirm the presence of distinct functional compartments of cAMP signaling in smooth muscles [23] as well as the ability of KIT2014 to act on selected subcellular cAMP pools [12, 19]. The present results, indicating that KIT2014 specifically blunted PDE4D function and abolished VSMC proliferation in response to PDGF and forskolin, conclusively demonstrated that, in the context of VSMC, the cAMP elevation elicited by KIT2014 is sufficient to reduce proliferation and IH.

Previous studies indicate that blocking PI3Kγ activity in VSMC could represent a new therapeutic approach to treat IH [24, 25]. However, PI3Kγ is a bifunctional protein endowed not only with the ability to interact with PKA and modulate cAMP levels (scaffold activity) but also to generate lipid second messenger molecules through its catalytic activity. Differently from what reported here, previous studies on PI3Kγ in VSMC focused on the catalytic role of the protein rather than its ability to interact with PKA and modulate cAMP levels. Yu et al. [25] explored the role of PI3Kγ catalytic activity in IH of arterial grafts and in TNF-α-induced phenotypic switch of VSMC. This study teaches that inhibition of PI3Kγ catalytic activity with the selective inhibitor AS605240 as well as shRNA-mediated knockdown of PI3Kγ result in the same protective effects that overall reduce IH in a model of aortic restenosis [25]. Both pharmacological and genetic inhibition of PI3Kγ led to reduced Akt phosphorylation, a well-established marker of PI3K catalytic activity. As an enzyme, PI3Kγ generates the second messenger molecule PIP3, that in turn is essentially required to mediate Akt phosphorylation. The finding that knockdown of the PI3Kγ gene provides the same result of the treatment with the selective inhibitor of the PI3Kγ kinase activity, let Yu et al. conclude that the key driver in IH is the ability of PI3Kγ to trigger Akt phosphorylation through its kinase activity and the ability to produce PIP3. Although PI3Kγ possesses a scaffold function unrelated to its kinase activity and indirectly linked to the regulation of the other secondary messenger molecule cAMP, this is not mentioned by Yu et al. [25] that explain the observed effects through the modulation of the catalytic activity only.

Given that the expression of PI3Kγ occurs not only in VSMC but also in leukocytes that can indirectly modulate VSMC responses and IH, the specific involvement of each of these two cell types was explored in models of PI3Kγ-dependent IH [24]. In this second study, Yu et al. first confirm that the kinase activity of PI3Kγ is involved in IH in response to vascular injury. In their model they corroborate that both pharmacological inhibition and genetic knockdown of PI3Kγ reduce IH. They report that reduction of the catalytic activity of PI3Kγ blocks phosphorylation and activation of Akt. In line with this effect, they also demonstrate that Akt-mediated phosphorylation of CREB as well as CREB-dependent transcriptional activation of YAP are determined by the catalytic activity of PI3Kγ. In addition, to address the question of the specific cell type involved in this process, they show that specific downregulation of PI3Kγ in either leukocytes or vascular tissue concur to the protection against IH development in response to vascular injury [24]. All these studies converge on the catalytic activity of PI3Kγ and fail to explore whether the scaffold function of this protein and cAMP levels are involved.

On the contrary, data presented herein clearly demonstrate that the kinase independent function of PI3Kγ affects proliferation of VSMC through a direct, cell-autonomous mechanism. The present data conclusively discriminate between a kinase-dependent and a kinase-independent role of PI3Kγ *in vivo* through the study of mice genetically engineered to express a kinase-dead version of the enzyme (PI3Kγ-KD mice) that retains the ability to interact with PKA [19]. The present results with PI3Kγ-KD mice clearly demonstrated that loss of the catalytic activity in the hematopoietic compartment led to a small reduction of IH. Conversely, loss of the PI3Kγ catalytic activity in the non-hematopoietic compartment was not able to block IH. In line with a role of the protein-protein interaction between PI3Kγ and PKA, complete loss of PI3Kγ in KO mice led to a strong in vivo reduction of IH. As a further prove of the involvement of the docking function of PI3Kγ in the control of VSMC proliferation, a peptide specifically targeting the scaffold but not the catalytic activity of PI3Kγ (KIT2014) inhibits VSMC proliferation *in vitro.* In line with this view, the ability of KIT2014 to block VSMC proliferation acts independently of PI3Kγ catalytic activity and confirms that the two roles of the enzyme can be independently targeted.

In summary, the present results are consistent with an unprecedented role for KIT2014 in disrupting the PI3Kγ/PKA complex promoting the PDE4D-dependent cAMP suppression required to maintain VSMC proliferation. On this basis, the inventors' findings point to a non-obvious use of KIT2014 in the treatment of IH, occurring in disease like restenosis, atherosclerosis and pulmonary arterial hypertension. KIT2014 can be used as a component of medicated stents or local treatments concomitant with angioplasty for the treatment of atherosclerosis and restenosis, or as an inhaled agent for the treatment of pulmonary arterial hypertension.

### Results

Inventors' previous results identified a role of the catalytic activity of PI3Kγ in immune processes of IH development and arterial healing [26]. This was demonstrated by using knock-in mice expressing a catalytically inactive PI3Kγ (PI3Kγ-KD) subjected to arterial mechanical injury. PI3Kγ KD mice showed reduced arterial occlusion and accumulation of monocytes and T cells around sites of vascular lesion. In the present study the inventors intended to further explore the implication of PI3Kγ in the vascular compartment, and to evaluate a possible role of its non-catalytical and docking function in IH. Bone marrow [27] chimeras were generated using PI3Kγ-KO and PI3Kγ-KD mice as recipients, to discriminate between kinase-dependent or kinase-independent function of PI3Kγ in the vascular compartment. The inventors introduced BM from WT into PI3Kγ-KO (WT>KO) or PI3Kγ-KD (WT>KD) animals and vice versa (KO>WT and KD>WT). After engraftment, femoral arteries were injured, and lesions were analyzed 28 days later. In BM WT>KO and KO>KO chimeras, the intima/media ratio was decreased by 40% compared to BM WT>WT chimera (Figure 1A), whereas no difference was observed in BM WT>KD chimeras as previously observed [26]. These results indicated that the presence of PI3Kγ, and not its catalytic activity, is required in the vascular compartment to induce a fibroproliferative response to the injury.

Consistently with the inventors' previous results [26], BM KO>WT, BM KD>WT or BM KD>KD chimeras showed a large decrease in intima/media ratio confirming the role of PI3Kγ catalytic activity in the immune compartment (Figure 1A). Histological analysis by Masson Trichrome staining of the injured arteries showed a decreased neointima area in BM WT>KO and BM KO>WT chimeras compared to controls (Figure 1A). This staining clearly indicated that the reduced neointima area in BM WT>KO is mostly due to a lower expansion of the VSMC layer (Figure 1A). Taken together, these results demonstrate that the post-injury VSMC proliferation is independent from the catalytic function of the PI3Kγ but rather involves its docking function.

Given the importance of cAMP in the inhibition of VSMC proliferation *in vitro* and *in vivo* [3-5], the inventors hypothesized that a kinase-independent function of PI3Kγ could also be implicated in VSMC proliferation *via* cAMP regulation. To test this hypothesis, the proliferation of primary VSMC from different genotypes (WT, PI3Kγ-KO or PI3Kγ-KD) was evaluated by stimulation with a powerful mitogenic agent for VSMC, platelet derived growth factor (PDGF). An adenylate cyclase activator, forskolin (FSK), was supplemented to culture media to increase cAMP levels and counterbalance the VSMC proliferation induced by PDGF (Figure 1C). The inventors' results showed that 25 µM of FSK decreased VSMC proliferation and showed that FSK effect is significantly potentiated in the absence of PI3Kγ (PI3Kγ-KO) but not in PI3Kγ-KD VSMC, confirming the role of AKAP function of PI3Kγ in VSMC (Figures 1D and E). These results demonstrated that PI3Kγ controls VSMC proliferation by regulating intracellular concentration of cAMP, independently from its enzymatic activity.

The involvement of the PI3Kγ in controlling intracellular concentration of cAMP in VSMC was confirmed by studying the dynamic of cAMP signals in WT, PI3Kγ-KD or PI3Kγ-KO primary VSMC using the FRET-based ^{T}Epac^{VV} biosensor [28]. Relative changes in cAMP levels in response to 2.5 µM forskolin were monitored over time, by calculating the ratio of donor fluorescence (F480nm) to acceptor fluorescence (F535nm). cAMP level is determined as a percentage of the maximum ratio change (Rmax). Rmax was determined at the end of the experiment by adding 200 µM 3-isobutyl-1-methylxanthine (IBMX), a pan-PDE inhibitor, in the presence of 2.5µM forskolin. As shown in figure 2, cAMP production in response to 2.5µM forskolin is increased in PI3Kγ-KO compared to WT primary VSMC by 2.2 folds (24.09% vs 52.57% of IBMX response, *p*=0.03), while forskolin response of PI3Kγ-KD cells remains similar to WT (Figure 2A-C). cAMP basal levels were not altered at steady states in all three genotypes (Figure 2A and B). Moreover, the maximum ratio change was also identical in VSMC from all genotypes (Figure 2A). Altogether, these results showed that the cAMP response to forskolin is higher in PI3Kγ-KO VSMC than in WT and PI3Kγ-KD cells, demonstrating a role for PI3Kγ in cAMP level limitation.

The intracellular level of cAMP depends on its production and degradation by adenylyl cyclases (AC) and PDE, respectively. PI3Kγ has already been shown to bind PDE in cardiomyocytes [11, 19], and among these the inventors focused on PDE3 and 4 since they have been implicated in IH [8, 10, 29]. They successively treated forskolin-stimulated VSMC with cilostamide and rolipram, inhibiting PDE3 and PDE4 respectively, and quantified cAMP level according to the Rmax. These experiments allowed to evaluate the relative proportion of cAMP pools regulated by each PDE in different genotypes. The results showed that cAMP levels are mostly regulated by PDE4 in WT and PI3Kγ-KD cells (67% and 73% respectively relatively to the IBMX response). PDE3 implication in cAMP levels regulation in these cells is limited to 25% and 17% of IBMX response, respectively (Figure 3A, B and C). Accordingly, in PI3Kγ-KO cells the major PDE isoform degrading cAMP is PDE3 (49 % of IBMX response for PDE3 vs 43% of IBMX response for PDE4, Figure 2C). To confirm the implication of PDE4 enzymes in cAMP regulation downstream of PI3Kγ, the inventors performed immunoprecipitation and measured PDE4 activity. They focused on PDE4D which is the main isoform expressed in VSMC. Consistent with inventors' cAMP measurements in living cells, PDE4D activity was significantly reduced in PI3Kγ-KO cells compared to WT and PI3Kγ-KD (Figure 3D and E).

PI3Kγ has been shown to regulate cAMP levels and signaling *via* its AKAP function in cardiomyocytes [19]. Interestingly, the interaction between PI3Kγ and the PKA/PDE complex has been mapped to the N-terminal region of p110γ between amino acid 126 and 150 [19]. To further investigate the functional relationship between PI3Kγ, cAMP regulation by PKA/PDE and VSMC proliferation, the inventors disrupted the PI3Kγ/PKA interaction by treating the cells with a cell penetrating competing peptide corresponding to the binding region of PKA on p110γ, and probed for their proliferation in response to PDGF and forskolin [19]. A control peptide has been designed with two-point mutations (K>A and R>A at position 1 and 5 of the PI3Kγ sequence, respectively) that does not interact with PKA [19] (Figure 4A). First, the inventors assessed the ability of the peptide to selectively interfere with the scaffold and not the catalytic function of PI3Kγ. They found that C5a-mediated Akt phosphorylation in macrophages, an event relying on the catalytic activity of PI3Kγ [30], was unaltered in cells treated with the PI3Kγ peptide, while significantly reduced in those exposed to the canonical PI3Kγ kinase inhibitor AS-6052540 (Figure 4B). Next, they determined the time course of peptide penetration in VSMC using a FITC-tagged version of the peptide. The peptide was detectable in cells after 5 hours and was still detectable 30h after incubation (Figure 4C-D). Thus, the inventors used the PI3Kγ peptide to block PI3Kγ-PKA interaction in an experiment of cell proliferation as in figure 1D. Incubation with the blocking peptide inhibited cell proliferation in WT and PI3Kγ-KD cells (Figure 5A and B) whereas no difference was observed in PI3Kγ-KO cells (Figure 5C). These data clearly demonstrate that PI3Kγ controls VSMC proliferation via its AKAP function.

Altogether, the present results allow to conclude that PI3Kγ acts as a catalyst of VSMC proliferation by decreasing cAMP levels mainly through the direct control of PDE4 enzymes (Figure 5D).

### Methods

**Animals:** PI3Kγ-deficient (PI3Kγ-KO) mice and mice expressing a catalytically inactive form of PI3Kγ (PI3Kγ kinase-dead mice; PI3Kγ-KD) were from a C57BL/6J background and have been described earlier [31-33]. WT, PI3Kγ-KD, PI3Kγ-KO were maintained at the animal facility of Rangueil (UMS 06; Anexplo platform) and kept under SPF conditions. All animal procedures were conducted in accordance with institutional guidelines on Animal Experimentation and were under a French Ministry of Agriculture license.

**Femoral artery wire injury in mice :** WT, PI3Kγ-KD, PI3Kγ-KO male mice aged 8-10 week were investigated using an established model of femoral artery wire injury [27], for IH studies. In brief, general anesthesia was achieved with 2% isoflurane. Then, the femoral artery was isolated, and an incision was made under a surgical microscope (Carl Zeiss). A 0.35-mm diameter angioplasty guide wire with a 0.25-mm tip was advanced three times into the artery up to the level of the aortic bifurcation then pulled back. After removal of the wire, the arteriotomy site was ligated. Mice were sacrificed 28 days after the injury for histological and immunohistochemical analysis.

**Tissue processing and morphometry:** IH quantification was performed by the paraffin-embedment technique. At the point of euthanasia, mice were perfused with PBS, followed by 4% paraformaldehyde. Vessels were harvested and fixed in 4% formalin, pH = 8, for 24 h. They were put into paraffin and prepared as slides (4-µm-thick sections). To quantify the rate of IH, sections were stained with Masson Trichrome (Bio Optica), and then the lumen, IEL (internal elastic lamina), and EEL (external elastic lamina) perimeters (µm) were measured at 4 sections (0.5, 2.0, 3.5, and 5 mm from the ligation site) for each vessel using LAS software (Leica). Arteries that underwent an occlusive thrombotic event were excluded from the quantification group. The area defined by the EEL (µm²) was calculated assuming circular geometry in vivo: (A_{EEL} = EEL circumference² ÷ 4π). The area defined by the IEL (µm²) was calculated using: (A_{IEL}= IEL circumference²÷ 4π). The lumen area (µm²) was calculated using: (A_{LUM} = Lumen circumference²÷ 4π). The neointimal area (µm²) was calculated using: (A_{NEO} = A_{IEL} - A_{LUM}). The medial area (µm²) was calculated using: (A_{MED} = A_{EEL} - A_{IEL}). The intima/media ratio was calculated using: (neointima/media = A_{NEO} ÷ A_{MED}).

**Bone Marrow (BM) transplantation:** BM was obtained from 8-week-old WT, PI3Kγ-KD and PI3Kγ-KO mice. BM cells were flushed from femurs and tibias, then washed, filtered, counted, and resuspended in sterile PBS for retro-orbital injection of 10⁷ unfractionated cells per WT, PI3Kγ-KD or PI3Kγ-KO irradiated mouse (9 Gy; Gilardoni RadGil irradiator). 4 weeks later, femoral artery injury was performed, and mice were euthanized 28 d after the surgery. The successful engraftment was confirmed by PCR.

**Isolation of SMC from murine aorta and culture:** Mouse VSMCs were isolated from WT mice according to a modified protocol described by [34]. In brief, aorta from 4 WT mice were dissected from their origin to the iliac bifurcation, flushed with PBS, and removed. The adventitia was removed from the aorta and the smooth tubes were cut into pieces of 1-2 mm, and then digested in 0.3% collagenase solution. Collagenase digestion was stopped by adding DMEM (D0822, Sigma Aldrich) /10% FBS (FBS, Thermo Fisher Scientific). Then, the pieces of aorta were washed twice and seeded in culture dishes. Primary confluent cultures were trypsinized (0.1% trypsin; Thermo Fisher Scientific) at 37°C, and then cells were incubated at 37°C in 5% CO₂ in DMEM/10% FBS and cultured until the fourth passage.

**Measure of mouse SMC proliferation:** Primary mouse SMCs were sub-cultured onto 48-well cell culture plates in complete medium. Cells were treated or not with PDGF (520-BB-050, R&D Systems) and forskolin (F3917, Sigma Aldrich) for 24h. For blocking peptide experiments, cells were incubated 24h with 25 µM of control (RQIKIWFQNRRMKWKKGAATHASPGQIHLVQRHPPSEESQAF - SEQ ID No.: 13) or blocking peptide (RQIKIWFQNRRMKWKKGKATHRSPGQIHLVQRHPPSEESQAF - SEQ ID No.: 14). Peptides were synthesized by GenScript (GenScript, Piscataway, NJ). BrdU (00-0103, Invitrogen) incorporation assays were performed by classical immunofluorescence procedure using anti-BrdU antibody (11-5071, eBioscience) and DAPI. Proliferation rate is measured as the ratio of BrdU positive nuclei over total nuclei and expressed as fold increase.

**Measurement of cAMP dynamics:** Primary SMC were seeded on coverslips and infected with the ^{T}Epac^{VV}-encoding type-5 adenovirus (~100 particles per SMC) as described elsewhere [35]. Coverslips were placed in a microscope chamber continually perfused (2mL·min⁻¹) with BBS buffer (125mM NaCl, 2mM CaCl₂, 1mM MgCl₂, 1.25mM NaH₂PO₄, 26mM NaHCO₃, 25mM glucose) saturated with 95% O₂-5% CO₂. Ratiometric analyses were performed as follows: fluorescence was excited with a LED source at 435nm and fluorescence emission was monitored with a dichroic mirror (T450LPXR) and alternating emission filters for the donor (HQ480/40) and acceptor (D535/40). Pairs of images were recorded with an Orca-ER CCD camera (Hamamatsu Photonics, Japan), at 20-s intervals. Changes in [cAMP]i are expressed as the ratio of donor fluorescence (F480) to acceptor fluorescence (F535). The ratios were multiplied by a same constant for all experiments, such that the baseline ratio was 1 in basal conditions. The maximum ratio change (Rmax) was obtained by stimulating cells with 2.5 µM forskolin and 200 µM IBMX (I5879, Sigma Aldrich). Filters and mirrors were obtained from AHF analysentechnik AG, Tübingen, Germany.

**Measurement of PDE activity:** Cells were scraped in 120 mmol/L NaCl, 50 mmol/L Tris-HCl (pH 8.0) and 1% Triton X-100, supplemented with protease and phosphatase inhibitors and centrifugated at 13000 rpm for 10 min at 4°C. Supernatants were subjected to immunoprecipitation and then assayed for PDE activity or Western Blotting. For immunoprecipitation assays, 200 µg of pre-cleared extracts were incubated with 20µl of a 1:1 slurry of protein A- or protein G-Sepharose (Amersham Biosciences, Buckinghamshire, UK) and 1µg of anti-PDE4D antibody (Abcam #ab171750) for 2 h at 4°C. Immunocomplexes were then extensively washed with lysis buffer and subjected to PDE activity assay. PDE activity in immunoprecipitates was measured according to the two-step method of Thompson and Appleman with minor modifications. In brief, immunoprecipitations were assayed in a total volume of 200 µl of reaction mixture containing 40 mmol/L Tris-HCl (pH 8.0), 1 mmol/L MgCl₂, 1.4 mmol/L 2-mercapto-ethanol, 1µmol/L cAMP (Sigma-Aldrich, Saint Louis, MO) and 0.1 µCi of [³H]cAMP (Amersham Bioscience, Buckinghamshire, UK) for 45 min at 33°C. To stop the reaction samples were boiled at 95°C for 3 min. The PDE reaction product 5'-AMP was then hydrolyzed by incubation of the assay mixture with 50 µg of Crotalus Atrox snake venom for 15 min at 37°C (Sigma-Aldrich, Saint Louis, MO). The resulting adenosine was separated by anion exchange chromatography using 400 µl of a 30% (w/v) suspension of Dowex AG1-X8 resin (Bio-Rad, Segrate, Milano, Italy). The amount of radiolabelled adenosine in the supernatant was quantitated by scintillation counting (Ultima Gold scintillation liquid from Perkin Elmer, Waltham, MA).

**C5a-mediated Akt phosphorylation in macrophages:** After 3 h starvation in serum-free medium, RAW264 macrophages were pre-treated with the PI3Kγ competing peptide (25 µM) or the PI3Kγ inhibitor AS-605240 (1µM; MedChemExpress) or DMSO for 30 min and stimulated for 5 min with 50 nM of C5a (Sigma Aldrich). Akt phosphorylation at Ser-473 was detected by Western blot using a Ser473 Akt-specific antibody (Cell Signaling Technology).

### References

1. Lacolley, P., V. Regnault, and A.P. Avolio, Smooth muscle cell and arterial aging: basic and clinical aspects. Cardiovasc Res, 2018. 114(4): p. 513-528.
2. Owens, G.K., M.S. Kumar, and B.R. Wamhoff, Molecular regulation of vascular smooth muscle cell differentiation in development and disease. Physiol Rev, 2004. 84(3): p. 767-801.
3. Bobin, P., et al., Cyclic nucleotide phosphodiesterases in heart and vessels: A therapeutic perspective. Arch Cardiovasc Dis, 2016. 109(6-7): p. 431-43.
4. Hewer, R.C., et al., PKA and Epac synergistically inhibit smooth muscle cell proliferation. J Mol Cell Cardiol, 2011. 50(1): p. 87-98.
5. Indolfi, C., et al., 8-chloro-cAMP inhibits smooth muscle cell proliferation in vitro and neointima formation induced by balloon injury in vivo. J Am Coll Cardiol, 2000. 36(1): p. 288-93.
6. Smith, S.A., A.C. Newby, and M. Bond, Ending Restenosis: Inhibition of Vascular Smooth Muscle Cell Proliferation by cAMP. Cells, 2019. 8(11).
7. Sassone-Corsi, P., The cyclic AMP pathway. Cold Spring Harb Perspect Biol, 2012. 4(12).
8. Begum, N., S. Hockman, and V.C. Manganiello, Phosphodiesterase 3A (PDE3A) deletion suppresses proliferation of cultured murine vascular smooth muscle cells (VSMCs) via inhibition of mitogen-activated protein kinase (MAPK) signaling and alterations in critical cell cycle regulatory proteins. J Biol Chem, 2011. 286(29): p. 26238-49.
9. Cai, Y., et al., Role of cAMP-phosphodiesterase 1C signaling in regulating growth factor receptor stability, vascular smooth muscle cell growth, migration, and neointimal hyperplasia. Circ Res, 2015. 116(7): p. 1120-32.
10. Tilley, D.G. and D.H. Maurice, Vascular smooth muscle cell phenotype-dependent phosphodiesterase 4D short form expression: role of differential histone acetylation on cAMP-regulated function. Mol Pharmacol, 2005. 68(3): p. 596-605.
11. Ghigo, A., et al., Phosphoinositide 3-kinase gamma protects against catecholamine-induced ventricular arrhythmia through protein kinase A-mediated regulation of distinct phosphodiesterases. Circulation, 2012. 126(17): p. 2073-83.
12. Hirsch, E. and A. Ghigo, Novel Pi3k Gamma Inhibitor Peptide For Treatment Of Respiratory System Diseases. 2016, KITHER BIOTECH S R L: WO 2016/103176.
13. Fougerat, A., et al., Key role of PI3Kgamma in monocyte chemotactic protein-1-mediated amplification of PDGF-induced aortic smooth muscle cell migration. Br J Pharmacol, 2012. 166(5): p. 1643-53.
14. Basatemur, G.L., et al., Vascular smooth muscle cells in atherosclerosis. Nat Rev Cardiol, 2019. 16(12): p. 727-744.
15. Newby, A.C. and A.B. Zaltsman, Molecular mechanisms in intimal hyperplasia. J Pathol, 2000. 190(3): p. 300-9.
16. Tuder, R.M., et al., Pathology of pulmonary hypertension. Clin Chest Med, 2007. 28(1): p. 23-42, vii.
17. Ishizaka, N., et al., Effects of a single local administration of cilostazol on neointimal formation in balloon-injured rat carotid artery. Atherosclerosis, 1999. 142(1): p. 41-6.
18. Ghigo, A., et al., PI3K and Calcium Signaling in Cardiovascular Disease. Circ Res, 2017. 121(3): p. 282-292.
19. Perino, A., et al., Integrating cardiac PIP3 and cAMP signaling through a PKA anchoring function of p110gamma. Mol Cell, 2011. 42(1): p. 84-95.
20. Estrada, B., et al., An integrated strategy for analyzing the unique developmental programs of different myoblast subtypes. PLoS Genet, 2006. 2(2): p. e16.
21. Moiseenko, A., et al., Origin and characterization of alpha smooth muscle actin-positive cells during murine lung development. Stem Cells, 2017. 35(6): p. 1566-1578.
22. Chao, Y.C., et al., Imaging cAMP nanodomains in the heart. Biochem Soc Trans, 2019. 47(5): p. 1383-1392.
23. Dekkers, B.G., K. Racke, and M. Schmidt, Distinct PKA and Epac compartmentalization in airway function and plasticity. Pharmacol Ther, 2013. 137(2): p. 248-65.
24. Yu, Q., et al., PI3Kgamma (Phosphoinositide 3-Kinase gamma) Regulates Vascular Smooth Muscle Cell Phenotypic Modulation and Neointimal Formation Through CREB (Cyclic AMP-Response Element Binding Protein)/YAP (Yes-Associated Protein) Signaling. Arterioscler Thromb Vasc Biol, 2019. 39(3): p. e91-e105.
25. Yu, Q., et al., PI3Kgamma promotes vascular smooth muscle cell phenotypic modulation and transplant arteriosclerosis via a SOX9-dependent mechanism. EBioMedicine, 2018. 36: p. 39-53.
26. Smirnova, N.F., et al., Targeting PI3Kgamma activity decreases vascular trauma-induced intimal hyperplasia through modulation of the Thl response. J Exp Med, 2014. 211(9): p. 1779-92.
27. Roque, M., et al., Mouse model of femoral artery denudation injury associated with the rapid accumulation of adhesion molecules on the luminal surface and recruitment of neutrophils. Arterioscler Thromb Vasc Biol, 2000. 20(2): p. 335-42.
28. Klarenbeek, J.B., et al., A mTurquoise-based cAMP sensor for both FLIM and ratiometric read-out has improved dynamic range. PLoS One, 2011. 6(4): p. e19170.
29. Inoue, Y., et al., Suppression of arterial intimal hyperplasia by cilostamide, a cyclic nucleotide phosphodiesterase 3 inhibitor, in a rat balloon double-injury model. Br J Pharmacol, 2000. 130(2): p. 231-41.
30. Camps, M., et al., Blockade of PI3Kgamma suppresses joint inflammation and damage in mouse models of rheumatoid arthritis. Nat Med, 2005. 11(9): p. 936-43.
31. Fougerat, A., et al., Genetic and pharmacological targeting of phosphoinositide 3-kinase-gamma reduces atherosclerosis and favors plaque stability by modulating inflammatory processes. Circulation, 2008. 117(10): p. 1310-7.
32. Hirsch, E., et al., Central role for G protein-coupled phosphoinositide 3-kinase gamma in inflammation. Science, 2000. 287(5455): p. 1049-53.
33. Patrucco, E., et al., PI3Kgamma modulates the cardiac response to chronic pressure overload by distinct kinase-dependent and -independent effects. Cell, 2004. 118(3): p. 375-87.
34. Ray, J.L., et al., Isolation of vascular smooth muscle cells from a single murine aorta. Methods Cell Sci, 2001. 23(4): p. 185-8.
35. Vallin, B., et al., Novel short isoforms of adenylyl cyclase as negative regulators of cAMP production. Biochim Biophys Acta Mol Cell Res, 2018. 1865(9): p. 1326-1340.

## Claims

1. A fusion peptide for use in treating, preventing and/or delaying onset of a fibroproliferative vascular disease, wherein the fusion peptide comprises:
(a) an amino acid sequence as defined in SEQ ID No.: 1 or a related homolog having at least 85% identity with SEQ ID No.: 1 and having the ability of the sequence SEQ ID No.: 1 to inhibit the kinase-independent function of PI3Kγ, and
(b) a peptide having the ability to penetrate a cell.

2. The fusion peptide for use according to claim 1, wherein the peptide having the ability to penetrate a cell is selected from the sequences specified in SEQ ID No.: 2 to 12.

3. The fusion peptide for use according to claim 1 or claim 2, wherein the peptide having the ability to penetrate a cell is linked to the C-terminal or the N-terminal of SEQ ID No.: 1 or its homologs.

4. The fusion peptide for use according to any one of the preceding claims, wherein the fusion peptide has an amino acid sequence as set forth in SEQ ID No.: 14.

5. The fusion peptide for use according to any one of the preceding claims, wherein the fibroproliferative vascular disease is selected from restenosis, hypertension, pulmonary hypertension, preferably pulmonary arterial hypertension, atherosclerosis and plaque rupture.

6. A pharmaceutical composition for use in treating, preventing and/or delaying onset of a fibroproliferative vascular disease containing a fusion peptide, and a pharmaceutically acceptable vehicle, wherein the fusion peptide comprises: (a) an amino acid sequence as defined in SEQ ID No.: 1 or a related homolog having at least 85% identity with SEQ ID No.: 1 and having the ability of the sequence SEQ ID No.: 1 to inhibit the kinase-independent function of PI3Kγ, and (b) a peptide having the ability to penetrate a cell.

7. The pharmaceutical composition for use according to claim 6, wherein the peptide having the ability to penetrate a cell is selected from the sequences specified in SEQ ID No.: 2 to 12.

8. The pharmaceutical composition for use according to claim 6 or claim 7, wherein the peptide having the ability to penetrate a cell is linked to the C-terminal or the N-terminal of SEQ ID No.: 1 or its homologs.

9. The pharmaceutical composition for use according to any one of claims 6 to 8, wherein the fusion peptide has an amino acid sequence as set forth in SEQ ID No.: 14.

10. The pharmaceutical composition for use according to any one of claims 6 to 9, wherein the fibroproliferative vascular disease is selected from restenosis, hypertension, pulmonary hypertension, preferably pulmonary arterial hypertension, atherosclerosis and plaque rupture.

## Patentansprüche

1. Fusionspeptid zur Verwendung bei der Behandlung, Prävention und/oder Verzögerung des Beginns einer fibroproliferativen Gefäßerkrankung, wobei das Fusionspeptid Folgendes umfasst:
(a) eine Aminosäuresequenz wie in SEQ ID Nr.: 1 definiert oder ein verwandtes Homolog, das mindestens 85% Identität mit SEQ ID Nr.: 1 aufweist und die Fähigkeit der Sequenz SEQ ID Nr.: 1 hat, die kinaseunabhängige Funktion von PI3Kγ zu hemmen, und
(b) ein Peptid, das die Fähigkeit hat, in eine Zelle einzudringen.

2. Fusionspeptid zur Verwendung nach Anspruch 1, wobei das Peptid, das die Fähigkeit hat, in eine Zelle einzudringen, aus den in SEQ ID Nr.: 2 bis 12 angegebenen Sequenzen ausgewählt ist.

3. Fusionspeptid zur Verwendung nach Anspruch 1 oder Anspruch 2, wobei das Peptid, das die Fähigkeit hat, in eine Zelle einzudringen, mit dem C-Terminus oder dem N-Terminus von SEQ ID Nr.: 1 oder deren Homologen verbunden ist.

4. Fusionspeptid zur Verwendung nach einem der vorhergehenden Ansprüche, wobei das Fusionspeptid eine Aminosäuresequenz wie in SEQ ID Nr.: 14 dargelegt aufweist.

5. Fusionspeptid zur Verwendung nach einem der vorhergehenden Ansprüche, wobei die fibroproliferative Gefäßerkrankung ausgewählt ist aus Restenose, Hypertonie, pulmonaler Hypertonie, vorzugsweise pulmonal-arterieller Hypertonie, Atherosklerose und Plaqueruptur.

6. Pharmazeutische Zusammensetzung zur Verwendung bei der Behandlung, Prävention und/oder Verzögerung des Beginns einer fibroproliferativen Gefäßerkrankung, die ein Fusionspeptid enthält, und ein pharmazeutisch verträgliches Vehikel, wobei das Fusionspeptid Folgendes umfasst: (a) eine Aminosäuresequenz wie in SEQ ID Nr.: 1 definiert oder ein verwandtes Homolog, das mindestens 85% Identität mit SEQ ID Nr.: 1 aufweist und die Fähigkeit der Sequenz SEQ ID Nr.: 1 hat, die kinaseunabhängige Funktion von PI3Kγ zu hemmen, und (b) ein Peptid, das die Fähigkeit hat, in eine Zelle einzudringen.

7. Pharmazeutische Zusammensetzung zur Verwendung nach Anspruch 6, wobei das Peptid, das die Fähigkeit hat, in eine Zelle einzudringen, aus den in SEQ ID Nr.: 2 bis 12 angegebenen Sequenzen ausgewählt ist.

8. Pharmazeutische Zusammensetzung zur Verwendung nach Anspruch 6 oder Anspruch 7, wobei das Peptid, das die Fähigkeit hat, in eine Zelle einzudringen, mit dem C-Terminus oder dem N-Terminus von SEQ ID Nr.: 1 oder deren Homologen verbunden ist.

9. Pharmazeutische Zusammensetzung zur Verwendung nach einem der Ansprüche 6 bis 8, wobei das Fusionspeptid eine Aminosäuresequenz wie in SEQ ID Nr.: 14 dargelegt aufweist.

10. Pharmazeutische Zusammensetzung zur Verwendung nach einem der Ansprüche 6 bis 9, wobei die fibroproliferative Gefäßerkrankung ausgewählt ist aus Restenose, Hypertonie, pulmonaler Hypertonie, vorzugsweise pulmonal-arterieller Hypertonie, Atherosklerose und Plaqueruptur.

## Revendications

1. Peptide de fusion pour une utilisation dans le traitement, la prévention et/ou le retardement de l'apparition d'une maladie vasculaire fibroproliférative, où le peptide de fusion comprend :
(a) une séquence d'acides aminés telle que définie dans SEQ ID n° : 1 ou un homologue apparenté ayant une identité d'au moins 85% avec SEQ ID n° : 1 et ayant la capacité de la séquence SEQ ID n° : 1 à inhiber la fonction indépendante de la kinase de PI3Kγ ; et
(b) un peptide ayant la capacité de pénétrer dans une cellule.

2. Peptide de fusion pour une utilisation selon la revendication 1, dans lequel le peptide ayant la capacité de pénétrer dans une cellule est choisi parmi les séquences spécifiées dans SEQ ID n° : 2 à 12.

3. Peptide de fusion pour une utilisation selon la revendication 1 ou 2, dans lequel le peptide ayant la capacité de pénétrer dans une cellule est lié à l'extrémité C-terminale ou N-terminale de SEQ ID n° : 1 ou de ses homologues.

4. Peptide de fusion pour une utilisation selon l'une quelconque des revendications précédentes, dans lequel le peptide de fusion a une séquence d'acides aminés telle que présentée dans SEQ ID n° : 14.

5. Peptide de fusion pour une utilisation selon l'une quelconque des revendications précédentes, dans lequel la maladie vasculaire fibroproliférative est choisie parmi la resténose, l'hypertension, l'hypertension pulmonaire, de préférence l'hypertension artérielle pulmonaire, l'athérosclérose et la rupture de plaque.

6. Composition pharmaceutique pour une utilisation dans le traitement, la prévention et/ou le retardement de l'apparition d'une maladie vasculaire fibroproliférative contenant un peptide de fusion et un véhicule pharmaceutiquement acceptable, où le peptide de fusion comprend : (a) une séquence d'acides aminés telle que définie dans SEQ ID n° : 1 ou un homologue apparenté ayant une identité d'au moins 85% avec SEQ ID n° : 1 et ayant la capacité de la séquence SEQ ID n° : 1 à inhiber la fonction indépendante de la kinase de PI3Kγ, et (b) un peptide ayant la capacité de pénétrer dans une cellule.

7. Composition pharmaceutique pour une utilisation selon la revendication 6, dans laquelle le peptide ayant la capacité de pénétrer dans une cellule est choisi parmi les séquences spécifiées dans SEQ ID n° : 2 à 12.

8. Composition pharmaceutique pour une utilisation selon la revendication 6 ou 7, dans laquelle le peptide ayant la capacité de pénétrer dans une cellule est lié à l'extrémité C-terminale ou N-terminale de SEQ ID n° : 1 ou de ses homologues.

9. Composition pharmaceutique pour une utilisation selon l'une quelconque des revendications 6 à 8, dans laquelle le peptide de fusion a une séquence d'acides aminés telle que présentée dans SEQ ID n° : 14.

10. Composition pharmaceutique pour une utilisation selon l'une quelconque des revendications 6 à 9, dans laquelle la maladie vasculaire fibroproliférative est choisie parmi la resténose, l'hypertension, l'hypertension pulmonaire, de préférence l'hypertension artérielle pulmonaire, l'athérosclérose et la rupture de plaque.
